(19) Europäisches Patentamt
European Patent Office
Office européen des brevets

(11) Veröffentlichungsnummer : **0 139 859**
**B1**

(12) **EUROPÄISCHE PATENTSCHRIFT**

(45) Veröffentlichungstag der Patentschrift : **19.10.88**

(21) Anmeldenummer : **84108000.5**

(22) Anmeldetag : **09.07.84**

(51) Int. Cl.$^4$ : **C 07 H 15/26**, A 61 K 31/70// C07H15/04, C07H15/06, C07H15/14

(54) **1,4-Dihydropyridine, Verfahren zu ihrer Herstellung sowie ihre Verwendung in Arzneimitteln.**

(30) Priorität : **22.07.83 DE 3326384**

(43) Veröffentlichungstag der Anmeldung : **08.05.85 Patentblatt 85/19**

(45) Bekanntmachung des Hinweises auf die Patenterteilung : **19.10.88 Patentblatt 88/42**

(84) Benannte Vertragsstaaten : **AT BE CH DE FR GB IT LI NL SE**

(56) Entgegenhaltungen :
**EP-A- 0 002 208**
**FR-A- 2 364 927**

(73) Patentinhaber : **BAYER AG**
**Konzernverwaltung RP Patentabteilung**
**D-5090 Leverkusen 1 Bayerwerk (DE)**

(72) Erfinder : **Heiker, Fred Robert, Dr.**
**Pahlkestrasse 15**
**D 5600 Wuppertal (DE)**
Erfinder : **Stoltefuss, Jürgen, Dipl.Ing.**
**Parkstrasse 20**
**D 5657 Haan (DE)**
Erfinder : **Franchkowiak, Gerhard, Dr.**
**Henselweg 10**
**Wuppertal 1 (DE)**
Erfinder : **Schramm, Matthias, Dr.**
**Paffrather Strasse 38**
**D 5000 Koeln 80 (DE)**
Erfinder : **Thomas, Günter, Dr.**
**Claudiusweg 9**
**D 5600 Wuppertal 1 (DE)**
Erfinder : **Gross, Rainer, Dr.**
**Platzhoffstrasse 23**
**D 5600 Wuppertal 1 (DE)**

**EP 0 139 859 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue 3-Nitro-1,4-dihydropyridin-Zuckerderivate, Verfahren zu ihrer Herstellung sowie ihre Verwendung in Arzneimitteln, insbesondere in kreislaufbeeinflussenden Arzneimitteln mit positiv inotroper Wirkung.

Es ist bereits bekannt geworden, daß 1,4-Dihydropyridine gefäßerweiternde Eigenschaften besitzen und als Coronarmittel und Antihypertensiva verwendet werden können (vgl. britisches Patent 1 173 062 ; britisches Patent 1 358 951 ; DE-OS 2 629 892 und DE-OS 2 752 820). Weiterhin ist bekannt, daß 1,4-Dihydropyridine als Calciumantagonisten eine Hemmung der Kontraktionskraft von glatten und kardialen Muskeln bewirken und zur Behandlung von Coronar- und Gefäßerkrankungen eingesetzt werden können (vgl. A. Fleckenstein, Ann. Rev. Pharmacol. Toxicol. 17, 149-166 (1977)).

Aus der Anmeldung FR-A-2 364 927 sind bereits Dihydropyridinderivate bekannt, die in der Esterfunktion einen Zuckerrest tragen. Die dort beschriebenen Verbindungen besitzen keine direkte Nitrosubstitution am Dihydropyridinring und zeigen calciumantagonistische bzw. gefäßerweiternde Wirkungen. Aus EP-A-2 208 sind bereits nitrosubstituierte Dihydropyridine bekannt, die sich jedoch von den erfindungsgemäßen Verbindungen durch das Fehlen eines Zuckerrestes in den Esterpositionen unterscheiden. Weiterhin wird in dieser europäischen Anmeldung ebenfalls die calciumantagonistische, d. h. gefäßerweiternde bzw. negativ inotrope Wirkung beschrieben.

Bei Kenntnis dieser Eigenschaften der Dihydropyridine war es nicht vorhersehbar, daß die im folgenden genannten Verbindungen aus dieser Stoffklasse keine kontraktionshemmende, sondern eine kontraktionskraftverstärkende, am Herzmuskel positiv inotrope Wirkung besitzen.

Die vorliegende Erfindung betrifft neue 1,4-Dihydropyridin-Derivate der allgemeinen Formel (I)

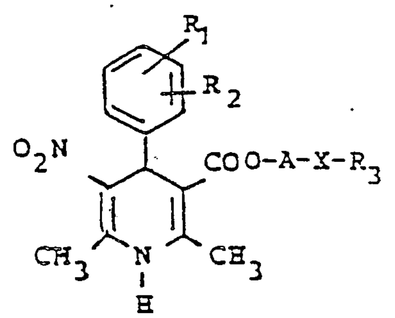

(I)

in welcher

$R_1$ und $R_2$ gleich oder verschieden sein können und für Wasserstoff, $C_1$-$C_4$-Alkyl, $C_1$ bis $C_{12}$-Alkoxy, $C_1$-$C_4$-Halogenalkoxy, Halogen, Nitro, $C_1$-$C_4$ Halogenalkyl, $C_1$-$C_4$ Halogenalkylmercapto oder eine der Gruppen

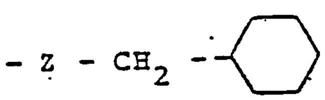

oder

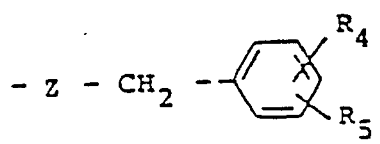

stehen,

in der Z Sauerstoff oder Schwefel bedeutet, und

$R_4$ und $R_5$ gleich oder verschieden sein können und Wasserstoff, $C_1$-$C_4$-Alkyl, $C_1$-$C_6$-Alkoxy, Halogen, $C_1$-$C_4$ Halogenalkyl, $C_1$-$C_4$ Halogenalkoxy oder Nitro bedeuten,

oder $R_1$ und $R_2$ gemeinsam mit 2 C-Atomen des Phenylringes den Ring

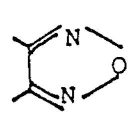

bilden,

X Sauerstoff, Schwefel oder den Rest $NR_6$, wobei $R_6$ eine $C_1$-$C_6$ Alkylgruppe bedeutet, darstellt

A eine $C_2$-$C_{10}$ Alkylengruppe darstellt, wobei mindestens 2 C-Atome in der Alkylenkette angeordnet sein müssen, die die Carboxylgruppe mit X verbindet

$R_3$ einen Pyranosyl oder Dipyranosylrest, der gegebenenfalls mit inder Kohlenhydratchemie üblichen

Schutzgruppen versehen ist, darstellt,
sowie ihre pharmazeutisch unbedenklichen Additionssalze.

Als Halogen seien bevorzugt Fluor, Chlor oder Brom, insbesondere Fluor oder Chlor genannt.

Der Rest $R_3$ stellt bevorzugt Pyranoseformen dar von : Hexosen wie Allose, Fructose, Sorbose, Altrose, Glucose, Mannose, Gulose, Idose, Galaktose, Talose ; Pentosen wie Ribose, Arabinose, Xylose und Lyxose ; Glucosamin ; Disaccharide wie Maltose, Lactose, Lactosamin, Cellobiose und Trehalose ; alle Gruppen gegebenenfalls mit den in der Kohlenhydratchemie üblichen Schutzgruppen versehen.

Insbesondere seien genannt : Hexosen wie Glucose, Mannose, Galaktose ; Pentosen wie Ribose, Xylose, Arabinose.

Als übliche Schutzgruppen werden vorzugsweise angeführt : Acylgruppen wie Acetyl oder Benzoyl ; säurespaltbare Äther wie Trityl oder Silyl ; Acetale und Ketale wie Isopropyliden, Cyclohexyliden, Benzyliden, Tetrahydropyranoyl ; Hydrogenolytisch abspaltbare Äther wie Benzyl.

Bevorzugte Verbindungen der Formel (I) sind diejenigen, in denen
$R_1$ für Wasserstoff steht,
$R_2$ Halogen, Trifluormethyl, Nitro, Wasserstoff oder eine der Gruppen

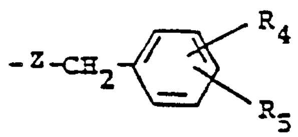 oder 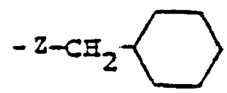

darstellt, wobei Z, $R_4$ und $R_5$ die bereits angegebenen Bedeutungen darstellen,
$R_3$ einen gegebenenfalls mit Schutzgruppen versehenen Pyranoserest darstellt,
X Sauerstoff oder Schwefel bedeutet und
A eine $C_2$-$C_6$ Alkylengruppe darstellt.

Die erfindungsgemäßen Dihydropyridine der allgemeinen Formel (I) können hergestellt werden, indem man

A) Aminocrotonsäureester der allgemeinen Formel (II)

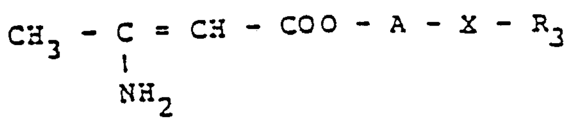 (II)

in welcher A, X und $R_3$ die oben angegebene Bedeutung haben, mit Aldehyden der allgemeinen Formel (III)

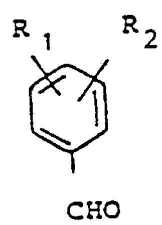 (III)

in welcher $R_1$ und $R_2$ die oben angegebene Bedeutung haben und Nitroaceton

$$CH_3—CO—CH_2—NO_2$$

umsetzt oder

B) Benzaldehyde der Formel (III) mit Acetessigsäureester der Formel (IV)

$$CH_3—CO—CH_2—COO—A—X—R_3 \qquad (IV)$$

in welcher A, X und $R_3$ die oben angegebene Bedeutung haben bzw. deren Knoevenagel-Kondensationsprodukte der Formel (V)

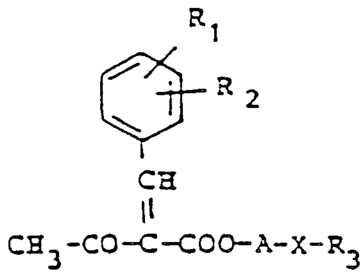

(V)

mit einer Additionsverbindung aus Nitroaceton und Ammoniak

$$CH_3—CO—CH_2—NO_2 \cdot NH_3$$

umsetzt, oder

C) indem man Aminocrotonsäureester der Formel (II) mit Benzylidennitroacetonen der Formel (VI)

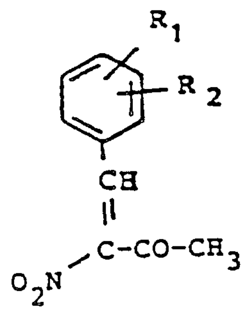

(VI)

umsetzt.

Bei den Herstellungsmethoden setzt man die Verbindungen der Formeln (II), (IV) und (V) im allgemeinen so ein, daß der Kohlenhydratsubstituent $R_3$ in geschützter Form vorliegt. Eine gegebenenfalls gewünschte Abspaltung der Schutzgruppe erfolgt nach üblichen Methoden, beispielsweise durch mit Basen oder Säuren katalysierte Ümesterung bei Acylschutzgruppen, säurekatalysierte Abspaltung von Acetalen, Ketalen, Trityl oder Silylgruppen sowie Hydrogenolyse von Benzylschutzgruppen.

Setzt man z. B. nach Verfahrensvariante A) β-Aminocrotonsäureester und Benzaldehyd mit Nitroaceton um, so läßt sich die Reaktion durch folgendes Formelschema wiedergeben :

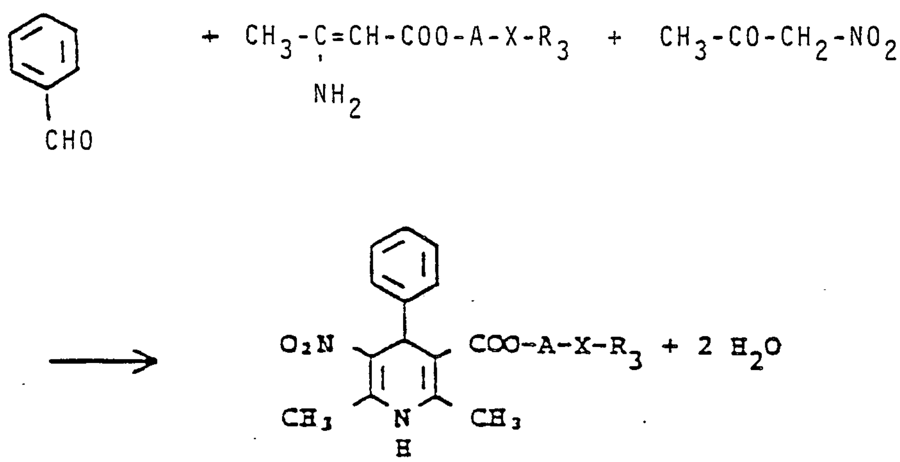

Setzt man z. B. nach Verfahrensvariante B) 2-Trifluorbenzyliden-acetessigsäureester mit Nitroaceton/Ammoniak um, so läßt sich die Reaktion durch folgendes Formelschema wiedergeben :

(Siehe Formelschema Seite 5 f.)

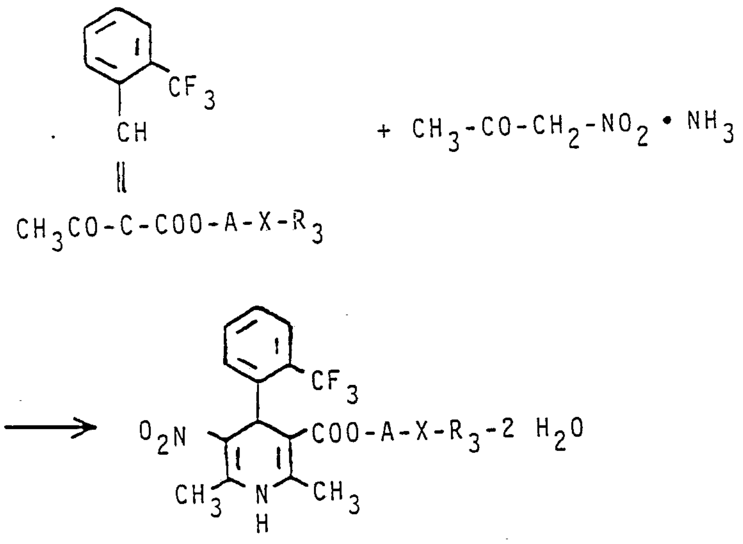

Setzt man z. B. nach Verfahrensvariante C) 2-Benzyloxybenzyliden-nitroaceton mit Aminocrotonsäureester um, so läßt sich die Reaktion durch folgendes Formelschema wiedergeben :

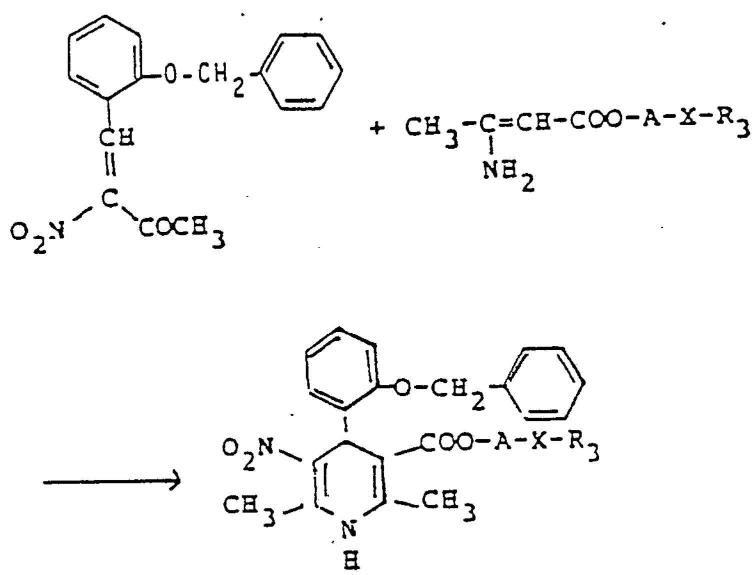

Für alle Verfahrensvarianten A, B und C kommen als Verdünnungsmittel alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150 °C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Die vorstehenden Herstellungsverfahren sind lediglich zur Verdeutlichung angegeben, und die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Das Mengenverhältnis der Reaktanden zueinander ist beliebig, im allgemeinen werden äquimolare Mengen eingesetzt. Es hat sich jedoch als zweckmäßig erwiesen, im Verfahren A Nitroaceton im bis zu 5 molaren Überschuß und im Verfahren B das Nitroaceton/Ammoniak-Addukt im bis zu 5 molaren Überschuß einzusetzen.

Je nach der Wahl der Ausgangssubstanzen können die erfindungsgemäßen Verbindungen in stereoisomeren Formen existieren, die sich nicht wie Bild und Spiegelbild (Diastereomere) verhalten. Sowohl die Diastereomeren als auch die Diastereomerengemische sind Gegenstand der vorliegenden

Erfindung. Die Diasteromerengemische lassen sich in bekannter Weise trennen (vgl. z. B. E.L. Eliel, Stereochemistry of Carbon Compounds, Mc Graw Hill, 1962).

Die Aminocrotonsäureester der Formel (II) sind nicht bekannt, können aber nach bekannten Methoden aus Acetessigestern der Formel (IV) mit Ammoniak hergestellt werden, vgl. A. C. Cope, J. Am. Chem. Soc. 67, 1017 (1945).

Die Acetessigester der Formel (IV) sind zum Teil bekannt oder können nach an sich bekannten Methoden hergestellt werden, indem man beispielsweise entweder

a) Furanosyl- oder Pyranosylhalogenide mit Verbindungen der Formel $HO—(CH_2)_n—XH$ reagieren läßt und das erhaltene Reaktionsprodukt mit Diketen zur Verbindung der Formel (IV) umsetzt, oder

b) Verbindungen der Formel (IV) mit X = S herstellt, indem man ω-Halogenalkohole zuerst mit Diketen umsetzt und den erhaltenen Acetessigsäure-ω-halogenalkylester mit 1-Thio-pyranosen oder -furanosen umsetzt, vgl. H. Paulsen, Angew. Chemie 94, 184-201 (1982) ; D. Borrmann, « Umsetzung von Diketen mit Alkoholen, Phenolen und Mercaptanen », in Houben-Weyl, Methoden der organischen Chemie, Vol. VII/4, 230 ff (1968) ; Y. Oikawa, K. Sugano und O. Yonemitsu, J. Org. Chem. 43, 2087 (1978).

Die verwendeten Aldehyde (III) sind bekannt oder können nach literaturbekannten Methoden dargestellt werden (vgl. T.D. Harris und G.P. Roth, J. org. Chem. 44, 146 (1979) ; Deutsche Offenlegungsschrift 2 165 260 ; Deutsche Offenlegungsschrift 2 401 665 ; Mijano et al., Chem. Abstr. 59 (1963), 13 929 c ; E. Adler und H.-D. Becker, Chem. Scand. 15, 849 (1961) ; E.P. Papadopoulos, M. Mardin und Ch. Issidoridis, J. Org. Chem. 31, 615 (1966), J. Am. chem. Soc. 78, 2543 (1956)).

Die erfindungsgemäß verwendbaren Yliden-β-carbonsäureester der Formel (V) sind nicht bekannt, können aber nach bekannten Methoden hergestellt werden [Organic Reactions XV, 204 ff (1967)].

Die einsetzbaren Verbindungen der Formel (VI) sind bekannt und beschrieben in H. Dornoff und W. Sassenberg, Liebigs Ann. Chem. 602, 14 (1957).

Das Nitroaceton x $NH_3$ Additionsprodukt kann analog H. Böhme und K.-H. Weise Arch. Pharm., 310, 30 (1977) hergestellt werden.

Nitroaceton läßt sich nach bekannten Methoden herstellen (vgl. N. Levy u. C.W. Scarfe, J. Chem. Soc. (London) (1946) 1103 ; C.D. Hurd and M.E. Nilson, J. Org. Chem. 20, 927 (1955)).

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt :

Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester), Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art

von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Die erfindungsgemäßen Verbindungen haben eine positive inotrope Wirkung und zeigen somit ein nicht vorhersehbares und wertvolles pharmakologisches Wirkungsspektrum. Sie können als Cardiotonika zur Verbesserung der Herzkontraktilität dienen. Darüber hinaus können sie als Antihypotonika, zur Senkung von Blutzucker, zur Abschwellung von Schleimhäuten und zur Beeinflussung des Salz- und Flüssigkeitshaushaltes eingesetzt werden.

Die positiv inotrope Wirkung der erfindungsgemäßen Verbindungen der Formel (I) wird in folgender Versuchsanordnung bestimmt ; wobei besonders solche Verbindungen bevorzugt sein sollen, die bereits ab einer Konzentration von $10^{-5}$ g/ml am linken Vorhof des isolierten Meerschweinchenherzens eine positiv inotrope Wirkung zeigen :

Die linken Vorhöfe von Meerschweinchenherzen werden isoliert und in ein thermostatisiertes Organbad gehängt, welches eine isotonische Mineralsalzlösung, die dem Ionenmilieu und dem pH-Wert von Körperflüssigkeiten angepaßt ist, mit geeigneten Nährstoffen enthält. Dieses Organbad wird mit einem Gasgemisch bestehend aus Sauerstoff und Kohlendioxid begast, wobei der Kohlendioxidgehalt so bemessen ist, daß der pH-Wert des Organbades konstant bleibt. Die linken Vorhöfe werden in das Organbad eingespannt, die Spannung wird mittels eines Kraftaufnehmers registriert, wobei ein bestimmter Grundtonus eingestellt wird. Anschließend werden die linken Vorhöfe kontinuierlich in bestimmten Abständen elektrisch gereizt und die dabei erfolgenden Kontraktionen registriert. Nach Zugabe des Wirkstoffs werden die Kontraktionen weiterhin registriert. Eine Kontraktionsverstärkung um mindestens 25 % gilt als signifikante positiv-inotrope Wirkung.

So werden zum Beispiel die Kontraktionen des mit 1 Hz elektrisch gereizten linken Meerschweinchenvorhofs durch $10^{-6}$ g/ml der Verbindung aus Beispiel 1 um 32 % und durch die Verbindung aus Beispiel 2 um 47 % verstärkt.

Weiterhin zeigen die erfindungsgemäßen Dihydropyridine eine bessere Löslichkeit im Vergleich zu den sehr schwer löslichen Dihydropyridinen aus dem Stand der Technik. Sie können somit in einfacherer Weise in galenische Zubereitungen gebracht werden, die eine verbesserte Bioverfügbarkeit besitzen.

Beispiel 1

1,4-Dihydro-2,6-dimethyl-3-nitro-4-(2-benzyloxy-phenyl)-pyridin-5-carbonsäure-2-O-(2,3,4,6-tetra-O-acetyl-β-D-glucopyranos-1-yl)-ethylester.

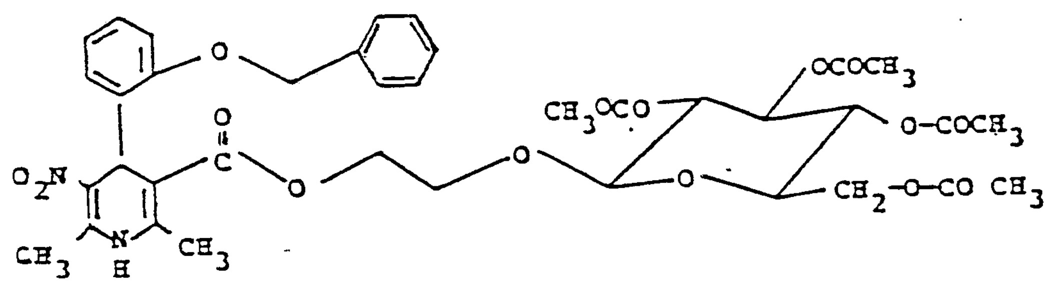

Verfahrensvariante A

2,7 g (12,5 mmol) 2-Benzyloxy-benzaldehyd werden mit 5,9 g (12,5 mmol) Aminocrotonsäure-2-O-(2,3,4,6-tetra-O-acetyl-β-D-glucopyranos-1-yl)-ethylester und 2,25 g (21,88 mmol) Nitroaceton in 50 ml Ethanol 3 1/2 Stunden zum Rückfluß erhitzt und anschließend eingeengt. Der erhaltene Eindampfrückstand wird über eine 35 cm lange Säule von 5 cm Durchmesser (Volumen 690 $cm^3$) mit Kieselgel 60, 0,04-0,063 mm (Merck) als stationärer Phase und Toluol/Essigester im Volumenverhältnis 6 : 1, später 4 : 1, getrennt. Die nahezu reinen Fraktionen werden vereinigt und eingeengt. Man erhält 3,7 g (39,3 % der Theorie) eines harzartigen gelben Produktes, welches beim Verreiben mit Ether kristallisiert. Es wird abgesaugt, mit Ether gewaschen und getrocknet. Man erhält 2,5 g (26,5 % der Theorie) gelbe Kristalle vom Schmelzpunkt 103-105 °C.

Beispiel 2

1,4-Dihydro-2,6-dimethyl-3-nitro-4-(2-benzyloxy)-phenylpyridin-5-carbonsäure-2-O-(β-D-glucopyranos-1-yl)-ethylester.

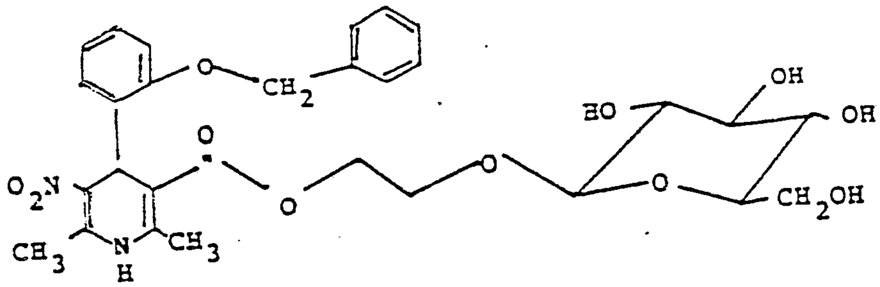

2,5 g (3,32 mmol) Verbindung aus Beispiel 1 werden in 25 ml absoluten Methanol gelöst und mit 1 ml 1 molarer Natriummethylatlösung unter Rühren versetzt, wobei die Farbe der Lösung von gelb in rot umschlägt. Nach 15 Minuten wird der Verlauf der Entblockierung durch Dünnschichtchromatographie überprüft. Da die Reaktion noch nicht beendet ist, werden weitere 0,5 ml 1 molarer Natriummethylatlösung zugesetzt. Nach 15 Minuten ist die Reaktion beendet. Es wird durch Zugabe von Amberlite-Ionenaustauscher IR 120 neutral gestellt, filtriert und eingeengt. Der kristalline Eindampfrückstand wird mit Ether/Methanol 3 : 1 verrührt, abgesaugt und mit eiskaltem Methanol und Ether gewaschen. Man erhält 1,7 g (92,14 % der Theorie) Produkt in Form gelber Kristalle (Schmelzpunkt 138-140 °C) Rf-Wert 0,37 DC-Alurolle, Kieselgel 60 F 254 (Merck) Fließmittel : Chloroform/Methanol im Volumenverhältnis 5 : 1.

Analog Beispiel 1 wurden erhalten :

Rf-Werte : DC-Alurolle Kieselgel 60 F 254 (Merck) ;

Fließmittel : Toluol/Ethanol im Volumenverhältnis 10 : 1.

Beispiel 3

1,4-Dihydro-2,6-dimethyl-3-nitro-4-[2-(4-methyl-benzyloxy)-phenyl]-pyridin-5-carbonsäure-2-O-(2,3,4,6-tetra-O-acetyl-β-D-glucopyranos-1-yl)-ethylester, isoliert als Schaum.

Rf-Wert : 0,3.

Beispiel 4

1,4-Dihydro-2,6-dimethyl-3-nitro-4-(2-benzyloxy-phenyl)-pyridin-5-carbonsäure-2-S-(2,3,4-tri-O-acetyl-1-thio-β-L-arabinopyranos-1-yl)-ethylester vom Schmelzpunkt 116-118 °C.

Beispiel 5

1,4-Dihydro-2,6-dimethyl-3-nitro-4-[2-(4-fluor-benzylmercapto)-phenyl]-pyridin-5-carbonsäure-3-S-(2,3,4,6-tetra-O-acetyl-1-thio-β-D-glucopyranos-1-yl)-propylester.

Rf-Wert : 0,295.

Beispiel 6

1,4-Dihydro-2,6-dimethyl-3-nitro-4-(2-benzyloxy-phenyl)-pyridin-5-carbonsäure-3-O-(2,3,4,6-tetra-O-acetyl-β-D-glucopyranos-1-yl)-n-propylester.

Rf-Wert : 0,305.

Beispiel 7

1,4-Dihydro-2,6-dimethyl-3-nitro-4-(2-benzyloxy-phenyl)-pyridin-5-carbonsäure-3-S-(2,3,4,6-tetra-O-acetyl-1-thio-β-D-glucopyranos-1-yl)-n-propylester.

Rf-Wert : 0,33.

Beispiel 8

1,4-Dihydro-2,6-dimethyl-3-nitro-4-(2-benzyloxy-phenyl)-pyridin-5-carbonsäure-2-S-(2,3,4,6-tetra-O-acetyl-1-thio-β-D-glucopyranos-1-yl)-ethylester.

RF-Wert : 0,315.

Beispiel 9

1,4-Dihydro-2,6-dimethyl-3-nitro-4-[2-(4-fluorbenzyloxy)-phenyl]-pyridin-5-carbonsäure-2-O-(2,3,4,6-tetra-O-acetyl-β-D-glucopyranos-1-yl)-ethylester.

Rf-Wert : 0,275.

Beispiel 10

1,4-Dihydro-2,6-dimethyl-3-nitro-4-(2-trifluormethylphenyl)-pyridin-5-carbonsäure-2-(2,3,4,6-tetra-O-acetyl-β-D-glucopyranos-1-yl)-ethylester.

Rf-Wert : 0,21.

Beispiel 11

1,4-Dihydro-2,6-dimethyl-3-nitro-4-phenyl-pyridin-5-carbonsäure-2-O-(2,3,4,6-tetra-O-acetyl-β-D-glucopyranos-1-yl)-ethylester.

Rf-Wert : 0,26.

Analog Beispiel 2 wurden hergestellt :

(Rf-Werte : DC-Alurolle Kieselgel 60, F 254 (Merck), Laufmittel : Chloroform/Methanol im Volumenverhältnis 5 : 1.

Beispiel 12

1,4-Dihydro-2,6-dimethyl-3-nitro-4-[2-(4-methyl-benzyloxy)-phenyl]-pyridin-5-carbonsäure-2-O-(β-D-glucopyranos-1-yl)-ethylester vom Schmelzpunkt 163 °C.

Beispiel 13

1,4-Dihydro-2,6-dimethyl-3-nitro-4-(2-benzyloxy-phenyl)-pyridin-5-carbonsäure-2-S-(1-thio-β-D-arabinopyranos-1-yl)-ethylester vom Schmelzpunkt 121 °C.

Beispiel 14

1,4-Dihydro-2,6-dimethyl-3-nitro-4-(2-benzyloxy-phenyl)-pyridin-5-carbonsäure-2-S-(1-thio-β-D-glucopyranos-1-yl) ethylester vom Schmelzpunkt 60-70 °C unter Zersetzung.

Beispiel 15

1,4-Dihydro-2,6-dimethyl-3-nitro-4-(2-benzyloxy-phenyl)-pyridin-5-carbonsäure-3-S-(1-thio-β-D-glucopyranos-1-yl)-n-propylester vom Schmelzpunkt 120-125 °C.

Beispiel 16

1,4-Dihydro-2,6-dimethyl-3-nitro-4-(2-benzyloxy-phenyl)-pyridin-5-carbonsäure-3-O-(β-D-glucopyranos-1-yl)-n-propylester.

Rf-Wert : 0,52

Beispiel 17

1,4-Dihydro-2,6-dimethyl-3-nitro-4-[2-(4-fluorbenzyloxy)-phenyl]-pyridin-5-carbonsäure-3-S-(1-thio-β-D-glucopyranos-1-yl)-n-propylester.

RF-Wert : 0,54.

Beispiel 18

1,4-Dihydro-2,6-dimethyl-3-nitro-4-[2-(4-fluorbenzyloxy)-phenyl]-pyridin-5-carbonsäure-2-O-(β-D-glucopyranos-1-yl)-ethylester.

Rf-Wert : 0,35.

Beispiel 19

1,4-Dihydro-2,6-dimethyl-3-nitro-4-phenyl-pyridin-5-carbonsäure-2-O-(β-D-glucopyranos-1-yl)-ethylester.

Rf-Wert : 0,25.

Beispiel 20

Verfahrensvariante C

1,06 g (5 mMol) 2-Benzyloxy-benzaldehyd werden mit 2,4 g (5 mMol) Acetessigsäure-2-O-(2,3,4,6-tetraacetyl-β-D-glucopyranosyl-ethylester und 0,9 g (7,5 mMol) Nitroaceton/Ammoniakaddukt im 10 ml

Ethanol 1 Stunde bei 60 °C gerührt. Anschließend wird 3 Stunden zum Rückfluß erhitzt. Der Ansatz wird eingeengt. Der Eindampfrückstand wird über eine 17 cm lange Säule von 4,5 cm Durchmesser (Volumen = 270 $cm^3$) mit Kieselgel 60, 0,04-0,063 mm (Merck) als stationärer Phase und Toluol Essigester im Volumenverhältnis 6 : 1, später 5 : 1, 4 : 1 und 3 : 1 getrennt. Das Produkt wird mit einem Volumenverhältnis von 1 : 1 erhalten. Die produktenthaltenden Fraktionen werden eingeengt. Man erhält 220 mg (5,8 % der Theorie) einer Verbindung, die identisch ist mit der Substanz aus Herstellungsbeispiel 1.

Beispiel 21

Verfahrensvariante B

1,75 g (6 mMol) einer Verbindung der Formel

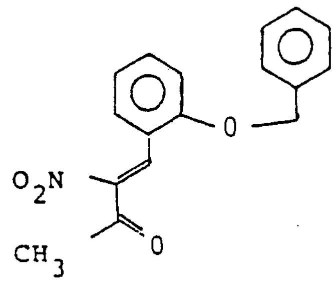

werden mit 2,6 g (6 mMol) Aminocrotonsäure-3-O-(2,3,4-tri-O-acetyl-α-L-arabinopyranos-1-yl)-propylester

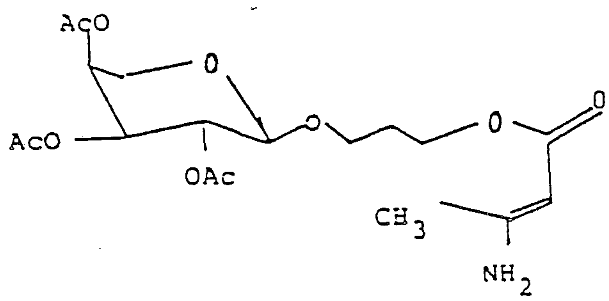

in 20 ml Ethanol über Nacht bei Rückfluß gerührt. Ansatz wird eingeengt und über ca. 50 g Kieselgel 230-400 Mesh in Toluol : Aceton 20 : 1 ; 10 : 1 getrennt. Man erhält 1,3 g (32 %).

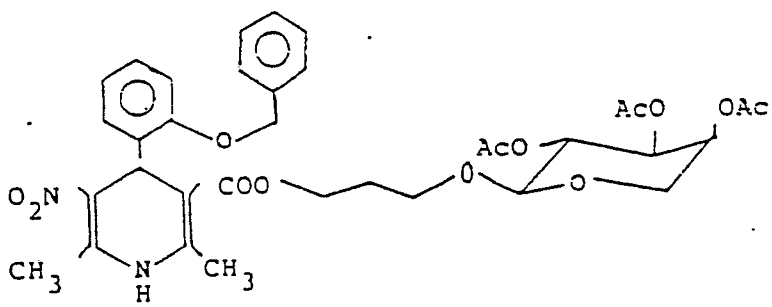

RF-Wert : 0,28 (Toluol/Aceton 4 : 1)
0,38 (Toluol/Ethanol 6 : 1)

Beispiel 22

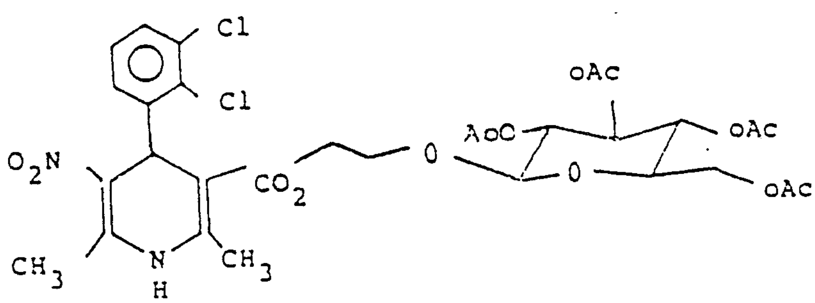

hergestellt nach Verfahrensvariante A
Rf-Wert : 0,2 (Toluol/Aceton 4 : 1)
0,34 (Toluol/Ethanol 6 : 1)

Beispiel 23

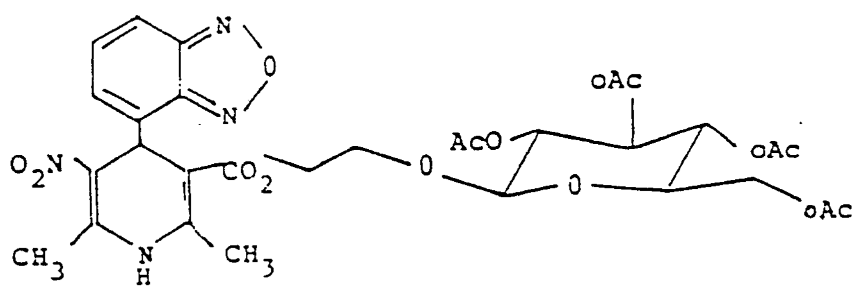

hergestellt nach Verfahrensvariante A
Rf-Wert : 0,24 (Toluol/Aceton 4 : 1)
0,34 (Toluol/Ethanol 6 : 1)

Herstellung der Ausgangsprodukte der Formel II bzw. IV

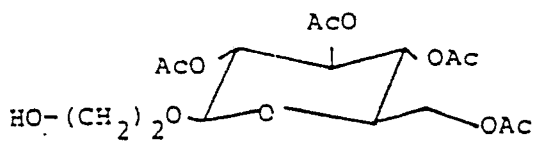

Ac = —$COCH_3$

A

48 g Acetobromglucose (117 mMol) wurden in 500 ml Toluol, 320 g Glykol und 57,6 g Silbercarbonat (208 mMol) 15 Stunden bei 20 °C gut gerührt und anschließend abgesaugt. Die Toluolphase wird abgetrennt und die Glykolphase 4 x mit Toluol extrahiert. Die vereinigten, getrockneten Phasen werden eingeengt und die Substanz aus isopropanol kristallisiert.

Man erhält 27 g rohes Produkt (59 % der Theorie).

Schmelzpunkt : 97-99 °C

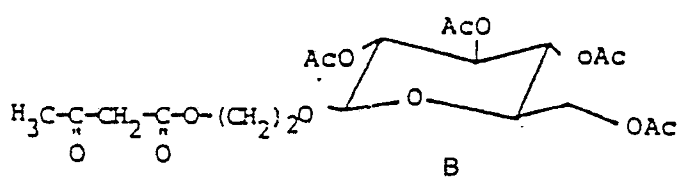

B

27 g A werden in 20 ml Toluol gelöst. Nach Zugabe von 1 ml Triethylamin wurden bei 80 °C 8,2 g Diketen zugetropft. Man rührt 1 Stunde bei 100 °C nach und gießt unter Rühren dann auf ein Gemisch aus 200 ml 5 % $NaHCO_3$ Lösung und 100 ml Toluol. Die Toluolphase wird abgetrennt, die Wasserphase mit Toluol nochmals ausgeschüttet und die org. Phasen nach dem Trocknen eingeengt. Man erhält nach Kristallisation aus Isopropanol 27,4 g reines Produkt (83,6 % der Theorie).

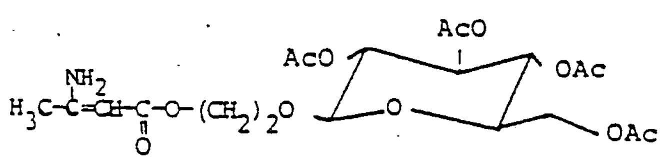

(II)

27,4 g B werden in 150 ml Toluol gelöst und unter Zugabe von 1 g p-Toluolsulfonsäure im Ammoniakstrom im Wasserabscheider 10 Stunden bei 110 °C gerührt. Nach Abkühlen, Absaugen und Einengen kristallisiert das Produkt aus Isopropanol aus. Man bekommt 19,7 g rohe Substanz (72 %) vom Schmelzpunkt 92-94 °C.

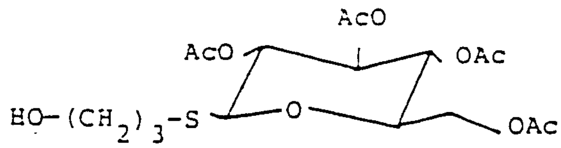

C

Zu 36,3 g (100 mMol) 1-S-2,3,4,6-tetra-O-acetyl-β-D-glucopyranose in 60 ml Aceton und 8,3 g $K_2CO_3$ in 100 ml $H_2O$ werden 16,7 g 3-Brompropanol-1 in 60 ml Aceton gegeben. Nach 2 Stunden rühren bei 20 °C werden 300 ml $CHCl_3$ und 100 ml Wasser zum Ansatz gegeben. Die org. Phase wird abgetrennt, die Wasserphase 1 x mit $CHCl_3$ extrahiert. Nun werden die vereinigten org. Phasen 1 x mit 2 N HCl und 1 x mit gesättigter Kochsalzlösung extr., getrocknet und eingeengt. Man erhält 42 g (100 %) C, welches zur weiteren Umsetzung rein genug ist.

**Patentansprüche**

1. 1,4-Dihydropyridin-Derivate der allgemeinen Formel (I)

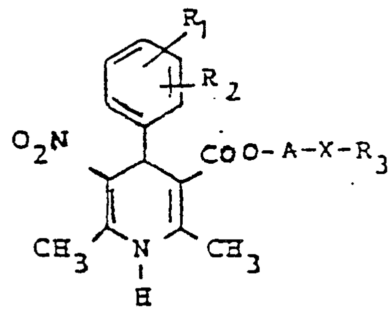

(I)

in welcher

$R_1$ und $R_2$ gleich oder verschieden sein können und für Wasserstoff, $C_1$-$C_4$-Alkyl, $C_1$ bis $C_{12}$-Alkoxy, $C_1$-$C_4$-Halogenalkoxy, Halogen, Nitro, $C_1$-$C_4$ Halogenalkyl, $C_1$-$C_4$-Halogenalkylmercapto oder eine der Gruppen

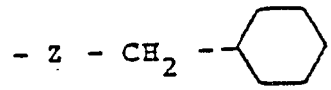

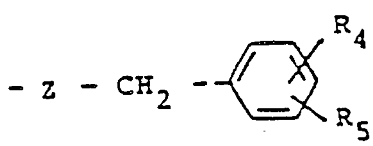

stehen,

in denen Z Sauerstoff oder Schwefel bedeutet, und

$R_4$ und $R_5$ gleich oder verschieden sein können und Wasserstoff, $C_1$-$C_4$-Alkyl, $C_1$-$C_6$-Alkoxy, Halogen, $C_1$-$C_4$ Halogenalkyl, $C_1$-$C_4$ Halogenalkoxy oder Nitro bedeuten,

oder $R_1$ und $R_2$ gemeinsam mit 2 C-Atomen des Phenylringes den Ring

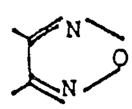

bilden,

X Sauerstoff, Schwefel oder den Rest $NR_6$, wobei $R_6$ eine $C_1$-$C_6$ Alkylgruppe bedeutet, darstellt,

A eine $C_2$-$C_{10}$ Alkylengruppe darstellt, wobei mindestens 2 C-Atome in der Alkylenkette angeordnet sein müssen, die die Carboxylgruppe mit X verbindet,

$R_3$ einen Pyranosyl oder Dipyranosylrest, der gegebenenfalls mit in der Kohlenhydratchemie üblichen Schutzgruppen versehen ist, darstellt,

sowie ihre pharmazeutisch unbedenklichen Additionssalze.

2. Verbindungen gemäß Anspruch 1, in denen

$R_1$ für Wasserstoff steht,

$R_2$ Halogen, Trifluormethyl, Nitro, Wasserstoff oder eine der Gruppen

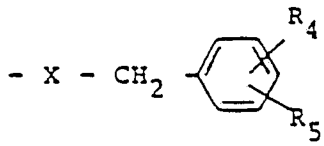 oder 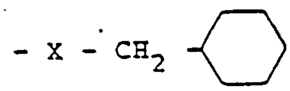

darstellt, wobei $R_4$ und $R_5$ die in Anspruch 1 genannten Bedeutungen aufweisen,

X Sauerstoff oder Schwefel bedeutet,

A eine $C_2$-$C_6$ Alkylengruppe darstellt und

$R_3$ einen gegebenenfalls mit Schutzgruppen versehenen Pyranosylrest darstellt.

3. Verbindungen gemäß Anspruch 1 und 2 zur Bekämpfung von Kreislauferkrankungen.

4. Verbindungen gemäß Anspruch 1 und 2 zur Verbesserung der Herzkontraktilität, zur Erhöhung des Blutdruckes, zur Senkung des Blutzuckers, zur Abschwellung von Schleimhäuten und/oder zur Beeinflussung des Salz- und Flüssigkeitshaushaltes.

5. Verfahren zur Herstellung der Verbindungen der allgemeinen Formel I

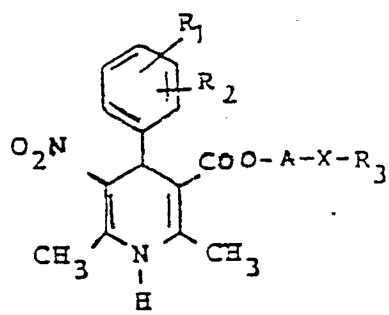

(I)

in welcher

$R_1$ und $R_2$ gleich oder verschieden sein können und für Wasserstoff, $C_1$-$C_4$-Alkyl, $C_1$ bis $C_{12}$-Alkoxy, $C_1$-$C_4$-Halogenalkoxy, Halogen, Nitro, $C_1$-$C_4$ Halogenalkyl, $C_1$-$C_4$-Halogenalkylmercapto oder eine der Gruppen

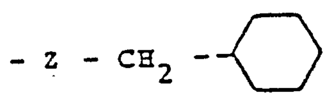

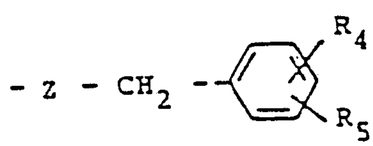

stehen,

in denen Z Sauerstoff oder Schwefel bedeutet, und

$R_4$ und $R_5$ gleich oder verschieden sein können und Wasserstoff, $C_1$-$C_4$-Alkyl, $C_1$-$C_6$-Alkoxy, Halogen, $C_1$-$C_4$ Halogenalkyl, $C_1$-$C_4$ Halogenalkoxy oder Nitro bedeuten,

oder $R_1$ und $R_2$ gemeinsam mit 2 C-Atomen des Phenylringes den Ring

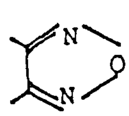

bilden,

X Sauerstoff, Schwefel oder den Rest $NR_6$, wobei $R_6$ eine $C_1$-$C_6$ Alkylgruppe bedeutet, darstellt,

A eine $C_2$-$C_{10}$ Alkylengruppe darstellt, wobei mindestens 2 C-Atome in der Alkylenkette angeordnet sein müssen, die die Carboxylgruppe mit X verbindet,

$R_3$ einen Pyranosyl oder Dipyranosylrest, der gegebenenfalls mit in der Kohlenhydratchemie üblichen Schutzgruppen versehen ist, darstellt,

sowie ihre pharmazeutisch unbedenklichen Additionssalze, dadurch gekennzeichnet, daß man

A) Aminocrotonsäureester der allgemeinen Formel (II)

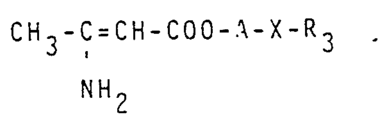

(II)

in welcher A, X und $R_3$ die in Anspruch 1 und 2 angegebene Bedeutung haben, mit Aldehyden der allgemeinen Formel (III)

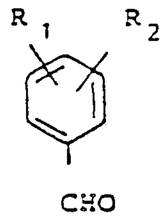

(III)

in welcher $R_1$ und $R_2$ die in Anspruch 1 und 2 angegebene Bedeutung haben und Nitroaceton

$$CH_3—CO—CH_2—NO_2$$

umsetzt oder

B) Benzaldehyde der Formel III mit Acetessigsäureester der Formel (IV)

$$CH_3—CO—CH_2—COO—A—X—R_3 \qquad (IV)$$

in welcher A, X und $R_3$ die in Anspruch 1 und 2 angegebene Bedeutung haben bzw. deren Knoevenagel-Kondensationsprodukte der Formel (V)

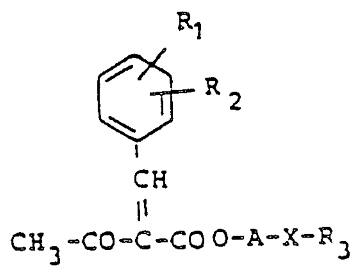

(V)

mit einer Additionsverbindung aus Nitroaceton und Ammoniak

$$CH_3—CO—CH_2—NO_2—NH_3$$

umsetzt oder,

C) Aminocrotonsäureester der Formel II mit Benzylidennitroacetonen der Formel VI

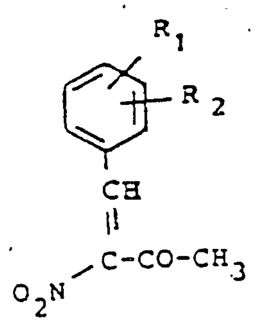

(VI)

umsetzt, und gegebenenfalls in ihre pharmazeutisch unbedenklichen Additionssalze überführt.

6. Verfahren gemäß Anspruch 5, dadurch gekennzeichnet, daß man ein Verdünnungsmittel einsetzt.

7. Verfahren gemäß Anspruch 5, dadurch gekennzeichnet, daß die Umsetzungen bei Temperaturen von 20 bis 150 °C durchgeführt werden.

8. Arzneimittel enthaltend mindestens eine Verbindung der Formel (I) gemäß Anspruch 1 und 2.

9. Verfahren zur Herstellung von Arzneimitteln, dadurch gekennzeichnet, daß man Verbindungen der Formel (I) gemäß Anspruch 5 gegebenenfalls unter Verwendung von üblichen Hilfs- und Trägerstoffen in eine geeignete Applikationsform überführt.

10. Verwendung von Verbindungen gemäß Anspruch 1 zur Herstellung von Arzneimitteln zur Verbesserung der Herzkontraktiliät, zur Erhöhung des Blutdrucks, zur Senkung von Blutzucker, zur Abschwellung von Schleimhäuten und zur Beeinflussung des Salz- und/oder Flüssigkeitshaushaltes.

**Claims**

1. 1,4-Dihydropyridine derivatives of the general formula (I)

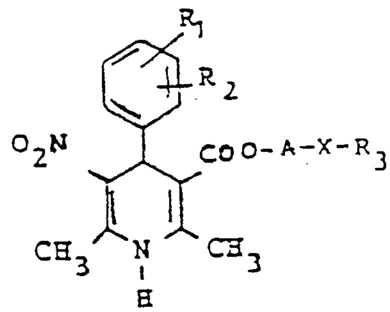

(I)

in which

$R_1$ and $R_2$ can be identical or different, and represent hydrogen, $C_1$-$C_4$-alkyl, $C_1$ to $C_{12}$-alkoxy, $C_1$-$C_4$-halogenoalkoxy, halogen, nitro, $C_1$-$C_4$-halogenoalkyl, $C_1$-$C_4$-halogeno-alkylmercapto or one of the groups

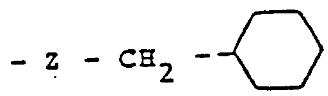

or

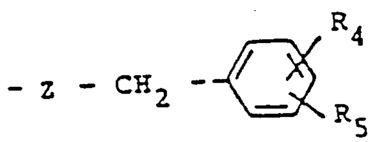

in which Z denotes oxygen or sulphur, and

$R_4$ and $R_5$ can be identical or different and denote hydrogen, $C_1$-$C_4$-alkyl, $C_1$-$C_6$-alkoxy, halogen, $C_1$-$C_4$-halogenoalkyl, $C_1$-$C_4$-halogenoalkoxy or nitro,

or $R_1$ and $R_2$, together with 2 C atoms of the phenyl ring, form the ring

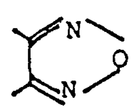

X represents oxygen, sulphur or the radical $NR_6$, $R_6$ denoting a $C_1$-$C_6$-alkyl group,

A represents a $C_2$-$C_{10}$-alkylene group, it being necessary that at least two C atoms are located in the alkylene chain which connects the carboxyl group to X, and

$R_3$ represents a pyranosyl or dipyranosyl moiety which is, where appropriate, provided with the protective groups customary in carbohydrate chemistry,

and their pharmaceutically acceptable addition salts.

2. Compounds according to Claim 1, in which

$R_1$ represents hydrogen,

$R_2$ represents halogen, trifluoromethyl, nitro, hydrogen or one of the groups

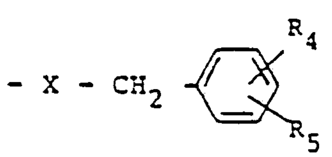

or

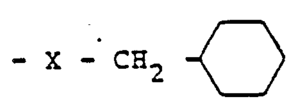

$R_4$ and $R_5$ having the meanings mentioned in Claim 1,

X denotes oxygen or sulphur,

A represents a $C_2$-$C_6$-alkylene group, and

$R_3$ represents a pyranosyl moiety which is, where appropriate, provided with protective groups.

3. Compounds according to Claim 1 and 2 for controlling cardiovascular diseases.

4. Compounds according to Claim 1 and 2 for improving the myocardial contractility, for increasing the blood pressure, for lowering the blood sugar, for reducing the swelling of mucous membranes and/or for affecting the salt and fluid balance.

5. Process for the preparation of the compounds of the general formula I

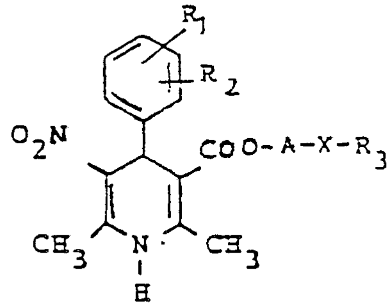

(I)

in which

$R_1$ and $R_2$ can be identical or different and represent hydrogen, $C_1$-$C_4$-alkyl, $C_1$ to $C_{12}$-alkoxy, $C_1$-$C_4$-halogenoalkoxy, halogen, nitro, $C_1$-$C_4$-halogenoalkyl, $C_1$-$C_4$-halogenoalkylmercapto or one of the groups

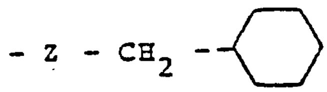

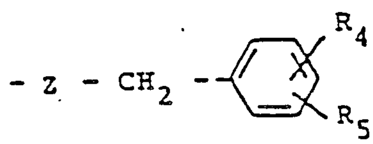

or

in which Z denotes oxygen or sulphur, and

$R_4$ and $R_5$ can be identical or different and denote hydrogen, $C_1$-$C_4$-alkyl, $C_1$-$C_6$-alkoxy, halogen, $C_1$-$C_4$-halogenoalkyl, $C_1$-$C_4$-halogenoalkoxy or nitro,

or $R_1$ and $R_2$, together with 2 C atoms of the phenyl ring, form the ring

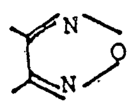

X represents oxygen, sulphur or the radical $NR_6$, $R_6$ denoting a $C_1$-$C_6$-alkyl group,

A represents a $C_2$-$C_{10}$-alkylene group, it being necessary that at least two C atoms are located in the alkylene chain which connects the carboxyl group to X, and

$R_3$ represents a pyranosyl or dipyranosyl moiety which is, where appropriate, provided with the protective groups customary in carbohydrate chemistry,

and their pharmaceutically acceptable addition salts, characterised in that

A) aminocrotonic esters of the general formula (II)

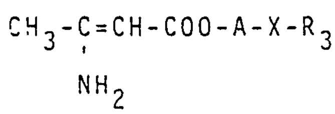

(II)

in which A, X and $R_3$ have the meaning indicated in Claim 1 and 2, are reacted with aldehydes of the general formula (III)

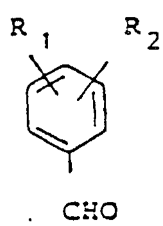

(III)

in which $R_1$ and $R_2$ have the meaning indicated in Claim 1 and 2, and nitroacetone

$$CH_3—CO—CH_2—NO_2$$

or

B) benzaldehydes of the formula III are reacted with acetoacetic esters of the formula (IV)

$$CH_3—CO—CH_2—COO—A—X—R_3 \qquad (IV)$$

in which A, X and $R_3$ have the meaning indicated in Claim 1 and 2, or their Knoevenagel condensation products of the formula (V)

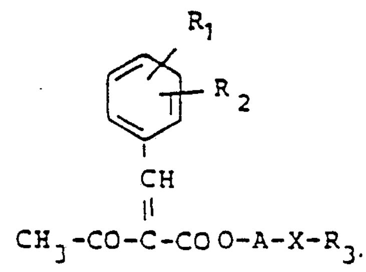

(V)

with an addition compound of nitroacetone and ammonia

$$CH_3—CO—CH_2—NO_2—NH_3$$

or

C) aminocrotonic esters of the formula II are reacted with benzylidenenitroacetones of the formula VI

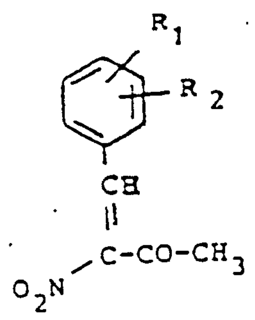

(VI)

and, where appropriate, are converted into their pharmaceutically acceptable addition salts.

6. Process according to Claim 5, characterised in that a diluent is employed.

7. Process according to Claim 5, characterised in that the reactions are carried out at temperatures from 20 to 150 °C.

8. Medicament containing at least one compound of the formula (I) according to Claim 1 and 2.

9. Process for the preparation of medicaments, characterised in that compounds of the formula (I) according to Claim 5 are converted into a suitable form for administration, optionally with the use of customary auxiliaries and vehicles.

10. Use of compounds according to Claim 1 for the preparation of medicaments for improving myocardial contractility, for increasing the blood pressure, for lowering the blood sugar, for reducing the swelling of mucous membranes and for affecting the salt and/or fluid balance.

**Revendications**

1. Dérivés de la 1,4-dihydropyridine, de formule générale (I)

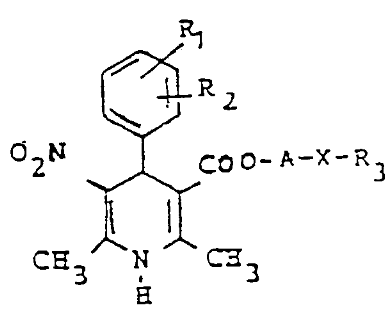

(I)

dans laquelle

$R_1$ et $R_2$ peuvent être identiques ou différents et ils représentent chacun un atome d'hydrogène, un groupe alkyle en $C_1$ à $C_4$, un groupe alcoxy en $C_1$ à $C_{12}$, un groupe halogénoalcoxy en $C_1$ à $C_4$, un atome d'halogène, un groupe nitro, un groupe halogénoalkyle en $C_1$ à $C_4$, un groupe halogénoalkylmercapto en $C_1$ à $C_4$ ou l'un des groupes

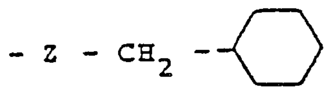

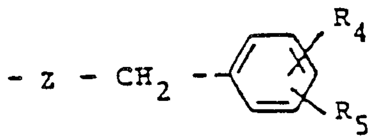

où Z représente un atome d'oxygène ou de soufre, et

$R_4$ et $R_5$ peuvent être identiques ou différents et ils représentent chacun un atome d'hydrogène, un groupe alkyle en $C_1$ à $C_4$, un groupe alcoxy en $C_1$ à $C_6$, un atome d'halogène, un groupe halogénoalkyle en $C_1$ à $C_4$, un groupe halogénoalcoxy en $C_1$ à $C_4$ ou un groupe nitro,

ou bien $R_1$ et $R_2$ forment ensemble, avec deux atomes de carbone du noyau phényle, le noyau

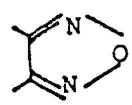

X représente un atome d'oxygène ou de soufre ou le reste $NR_6$, dans lequel $R_6$ représente un groupe alkyle en $C_1$ à $C_6$,

A représente un groupe alkylène en $C_2$ à $C_{10}$, la chaîne alkylène reliant le groupe carboxyle à X devant comporter au moins deux atomes de carbone,

$R_3$ représente un reste pyrannosyle ou dipyrannosyle, qui peut éventuellement comporter des groupes protecteurs usuels dans la chimie des glucides ou hydrates de carbone,

ainsi que leurs sels d'addition ne soulevant pas d'objection du point de vue pharmaceutique.

2. Composés selon la revendication 1, dans lesquels

$R_1$ représente un atome d'hydrogène,

$R_2$ représente un atome d'halogène, un groupe trifluorométhyle, nitro, un atome d'hydrogène ou l'un des groupes

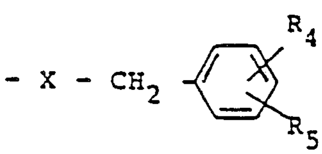

ou

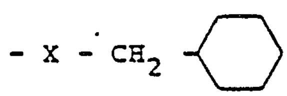

où $R_4$ et $R_5$ ont les sens indiqués à la revendication 1,

X représente un atome d'oxygène ou de soufre,

A représente un groupe alkylène en $C_2$ à $C_6$, et

$R_3$ représente un reste pyranosyle comportant éventuellement des groupes protecteurs.

3. Composés selon la revendication 1 et 2, pour lutter contre les maladies de la circulation sanguine.

4. Procédé selon la revendication 1 et 2 pour améliorer la contractivité cardiaque, pour élever la pression sanguine, pour diminuer la glycémie, pour provoquer la détumescence de muqueuses et/ou pour influer sur l'excrétion des sels et des liquides.

5. Procédé pour produire les composés de formule générale I

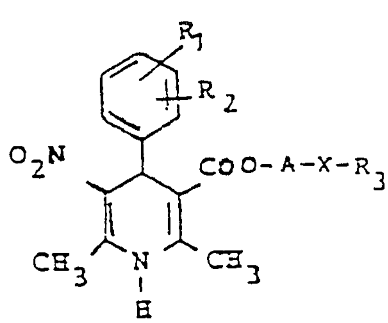

(I)

dans laquelle

$R_1$ et $R_2$ peuvent être identiques ou différents et ils représentent chacun un atome d'hydrogène, un groupe alkyle en $C_1$ à $C_4$, un groupe alcoxy en $C_1$ à $C_{12}$, un groupe halogénoalcoxy en $C_1$ à $C_4$, un atome d'halogène, un groupe nitro, un groupe halogénoalkyle en $C_1$ à $C_4$, un groupe halogénoalkylmercapto en $C_1$ à $C_4$, ou l'un des groupes

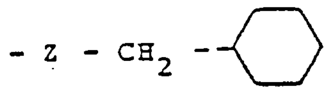

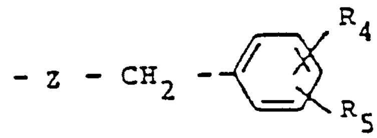

dans lesquels Z représente un atome d'oxygène ou de soufre, et

$R_4$ et $R_5$ peuvent être identiques ou différents et ils représentent chacun un atome d'halogène, un groupe alkyle en $C_1$ à $C_4$, un groupe alcoxy en $C_1$ à $C_6$, un atome d'halogène, un groupe halogénoalkyle en $C_1$ à $C_4$, un groupe halogénoalcoxy en $C_1$ à $C_4$ ou un groupe nitro,

ou bien $R_1$ et $R_2$ forment, avec deux atomes de carbone du noyau phényle, le noyau

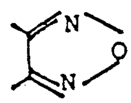

X représente un atome d'oxygène ou de soufre ou bien le reste $NR_6$, dans lequel $R_6$ représente un groupe alkyle en $C_1$ à $C_6$,

A représente un groupe alkylène en $C_2$ à $C_{10}$, ce groupe alkylène reliant le groupe carboxyle à X devant comporter au moins deux atomes de carbone,

$R_3$ représente un reste pyrannosyle ou dipyrannosyle, pouvant éventuellement comporter des groupes protecteurs usuels dans la chimie des glucides,

ainsi que de leurs sels d'addition ne présentant pas d'inconvénient du point de vue pharmaceutique, procédé caractérisé en ce que :

A) on fait réagir des esters de l'acide aminocrotonique de formule générale (II)

$$CH_3-\underset{NH_2}{\underset{|}{C}}=CH-COO-A-X-R_3 \quad (II)$$

dans laquelle A, X et $R_3$ ont le sens indiqué à la revendication 1 et 2, avec des aldéhydes de formule générale (III)

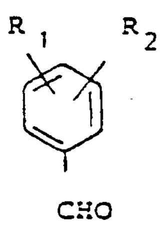

(III)

dans laquelle $R_1$ et $R_2$ ont le sens indiqué à la revendication 1 et 2, et avec la nitro-acétone

$$CH_3—CO—CH_2—NO_2$$

ou

B) on fait réagir des benzaldéhydes de formule III avec un ester de l'acide acéto-acétique de formule (IV)

$$CH_3—CO—CH_2—COO—A—X—R_3 \quad (IV)$$

dans laquelle A, X et $R_3$ ont le sens indiqué à la revendication 1 et 2, ou leurs produits de condensation de Knoevenagel, de formule (V)

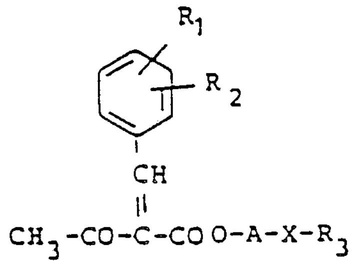

(V)

avec un composé d'addition de la nitro-acétone et de l'ammoniac

$$CH_3—CO—CH_2—NO_2—NH_3$$

ou

C) on fait réagir un ester d'acide aminocrotonique de formule II avec des benzylidène-nitro-acétones de formule VI

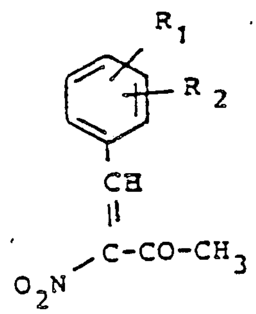

(VI)

et éventuelement on les transforme en leurs sels d'addition ne présentant pas d'inconvénient pharmaceutique.

6. Procédé selon la revendication 5, caractérisé en ce qu'on utilise un diluant.

7. Procédé selon la revendication 5, caractérisé en ce qu'on conduit les réactions à des températures de 20 à 150 °C.

8. Médicaments contenant au moins un composé de formule (I) selon la revendication 1 et 2.

9. Procédé pour préparer des médicaments, caractérisé en ce qu'on transforme des composés de formule (I) selon la revendication 5, en utilisant des adjuvants, supports et excipients usuels, en une forme convenant bien pour l'administration.

10. Utilisation de composés selon la revendication 1 pour la préparation des médicaments pour améliorer la contractilité cardiaque, pour élever la pression sanguine, pour diminuer la glycémie, pour provoquer la détumescence de muqueuses et pour influer sur l'excrétion de sel(s) et/ou de liquide(s).